# EUROPÄISCHE PATENTSCHRIFT

(11) **EP 1 179 179 B1**
(45) Veröffentlichungstag und Bekanntmachung des Hinweises auf die Patenterteilung: **20.10.2004**
(21) Anmeldenummer: 00929537.9
(22) Anmeldetag: 17.05.2000
(51) Int. Cl.: G01N 33/53, G01N 33/574, G01N 33/58, C12N 5/08

(54) **VERWENDUNG EINES ANTIKÖRPERS ZUR DETEKTION VON BASOPHILEN UND/ODER MASTZELLEN**
USE OF AN ANTIBODY TO DETECT BASOPHILES AND/OR MAST CELLS
UTILISATION D'UN ANTICORPS POUR DETECTER DES BASOPHILES ET/OU DES MASTOCYTES

(30) Priorität: 19.05.1999 DE 19922863; 12.06.1999 DE 19926879
(43) Veröffentlichungstag der Anmeldung: 13.02.2002
(73) Patentinhaber: Eberhard-Karls-Universität Tübingen Universitätsklinikum, 72076 Tübingen (DE)
(72) Erfinder: BÜHRING, Hans-Jörg, D-72076 Tübingen (DE); VAN AGTHOVEN, Andreas, Johannes, F-13009 Marseille (FR); JARROSSAY, David, CH-Bellinzona (CH)
(74) Vertreter: Otten, Hajo, Dr.-Ing.
(86) Internationale Anmeldenummer: PCT/EP2000/004468
(87) Internationale Veröffentlichungsnummer: WO 2000/072010

(56) Entgegenhaltungen:
- EP-A- 0 596 479
- EP-A- 0 863 157
- WO-A-97/46880
- WO-A-98/32014
- DE-C- 19 632 755
- CHEMICAL ABSTRACTS, vol. 127, no. 15, 13. Oktober 1997 (1997-10-13) Columbus, Ohio, US; abstract no. 202545, T. THOMAS ET AL.: "Antibody compositions for preparing enriched human hematopoietic and tumor cell preparations." Seite 376; Spalte 2; XP002149865 & CA 2 191 655 A (STEMCELL TECHNOLOGIES INC.) 2. Juni 1997 (1997-06-02)

## Beschreibung

Die Erfindung betrifft die Verwendung eines Antikörpers zur Detektion und/oder Quantifizierung und/oder Isolierung von Basophilen und/oder Mastzellen und/oder den Vorläuferzellen von Basophilen und/oder Mastzellen und/oder einer Oberflächenstruktur dieser Zellen.

Ferner betrifft die Erfindung
- ein Verfahren zur Untersuchung von Allergien, sowie
- ein Verfahren zur Bereitstellung von hämatopoetischen Vorläuferzellen, die in Mastzellen oder Basophile differenzieren können.

Antikörper, mit denen man Basophile und Mastzellen im ausgereiften Zustand und einzeln für sich detektieren kann, sind bekannt.

Bei der Hämatopoese oder Blutbildung gehen aus pluripotenten Stammzellen im Knochenmark lymphoide und myeloische Vorläuferzellen hervor. Aus den lymphoiden Vorläuferzellen entstehen T-und B-Lymphozyten, während aus den myeloischen Vorläuferzellen entweder Erythrozyten, Megakaryozyten, Basophile, Eosinophile, Neutrophile, Monozyten oder bisher unbekannte Vorläuferzellen, aus denen sich Mastzellen entwickeln, entstehen. Basophile, Eosinophile und Neutrophile werden insgesamt als Granulozyten bezeichnet. Nach der Hämatopoese finden sich Basophile im Blut, während sich Mastzellen in Geweben finden. Die Hämatopoese findet beim Erwachsenen im Knochenmark statt.

Basophile und Mastzellen sind multifunktionelle Effektorzellen, die an allergischen Reaktionen und an Entzündungsreaktionen beteiligt sind. Trotz ähnlicher biochemischer und funktioneller Eigenschaften sind Basophile und Mastzellen unterschiedliche Zelltypen, die beide ebenso wie Eosinophile von CD34-positiven Vorläuferzellen abstammen.

Die Unterscheidung der verschiedenen Zelltypen im Knochenmark und Blut sowie deren Zuordnung in verschiedene Differenzierungsstadien spielt im Klinikalltag eine wesentliche Rolle. So ist es beispielsweise zur Diagnose von Blutbildungsstörungen notwendig, die Zellzahl jedes spezifischen Zelltyps und möglichst auch das jeweilige Differenzierungsstadium zu erfassen.

Darüber hinaus ist die Untersuchung der Blut- und Blutvorläuferzellen im Knochenmark bei der Diagnose von Leukämien wichtig. Anzahl, Art und Stadium der Zellen werden zur Klassifizierung oder Zuordnung des Leukämietyps sowie zur Entscheidung über eine angemessene Therapie herangezogen. Dabei richtet sich die Zuordnung von Leukämien einerseits nach dem klinischen Verlauf der Krankheit und andererseits nach dem Reifegrad und der Abstammung der pathologisch veränderten Leukozyten. Dazu ist es jedoch notwendig, Zelltyp und Status sowohl der gesunden als auch der entarteten in einer Patientenprobe enthaltenen Zellen zu erfassen.

Bisher erfolgen diese Analysen im Mikroskop anhand der Morphologie der Zellen nach Anfärbung mit klassischen Färbemethoden, beispielsweise der Pappenheim-Färbung oder der May-Grünwald-Giemsa-Färbung, und durch manuelles Auszählen. Moderne Verfahren zur Auswertung von Knochenmarkbiopsien oder Blutproben verwenden Antikörper, die spezifische Antigene als Marker für bestimmte Zelltypen und Stadien erkennen. Die Antikörper bzw. die erkannten Antigene können dann in Standardverfahren wie dem ELISA (enzyme linked immunosorbent assay) oder der Durchflußzytometrie (FACS = fluorescence activated cell sorting) automatisiert nachgewiesen werden.

Bisher sind aber nur wenige Antikörper bekannt, die spezifisch intakte Basophile erkennen. Einer dieser Antikörper ist der Antikörper Bsp-1, der mit Basophilen aber nicht mit Gewebe-Mastzellen reagiert (Bodger, M.P. et al., Blood 69 (1987), 1414). Der CD117-reaktive Antikörper YB5B8 erkennt Mastzellen und hämatopoetische Vorläuferzellen (Ashman et al., Blood 78 (1991), 30).

In der DE 197 08 877 C1 ist beschrieben, daß der Antikörper 97A6 spezifisch an Megakaryozyten, nicht jedoch an Thrombozyten bindet.

Bisher ist jedoch von keinem Antikörper bekannt, daß er gleichzeitig Basophile, Mastzellen und deren Vorläuferzellen erkennt.

Vor diesem Hintergrund ist es Aufgabe der vorliegenden Erfindung, einen Antikörper für die eingangs genannte Verwendung bereitzustellen.

Diese Aufgabe wird erfindungsgemäß durch eine Verwendung eines Antikörpers gelöst, bei der eine Bindung des Antikörpers an die Oberflächenstruktur der Zellen erfolgt, an die der Antikörper mit der Bezeichnung 97A6 binden kann, der von Hybridomzellen produziert und freigesetzt wird, die am 12.02.1997 unter der Nr. DSM ACC 2297 bei der Deutschen Sammlung von Mikroorganismen und Zellkulturen GmbH, DSMZ, gemäß dem Budapester Vertrag hinterlegt worden sind.

Die Aufbewahrungsdauer dieser Hybridomzellen wurde entsprechend verlängert.

Unter einem Antikörper im Sinne der Erfindung werden auch Antikörperfragmente, wie beispielsweise F(ab), Konjugate mit Antikörpern und/oder Antikörperfragmenten sowie alle Zusammensetzungen, die Antikörper, Antikörperfragmente und Konjugate mit Antikörpern und/oder Antikörperfragmenten enthalten, verstanden.

Eine Oberflächenstruktur, an die ein Antikörper bindet, kann im Sinne der Erfindung ein einzelnes Molekül, beispielsweise ein Membranprotein, oder auch eine Assoziierung von zwei oder mehreren Molekülen, beispielsweise ein aus mehreren Untereinheiten bestehender Ionenkanal oder Rezeptor, sein.

Die der Erfindung zugrunde liegende Aufgabe wird auf diese Weise vollkommen gelöst.

Die Erfinder der vorliegenden Anmeldung haben nämlich erkannt, daß die Oberflächenstruktur, an die der Antikörper 97A6 binden kann, außer auf wenigen megakaryozytischen Zellinien nur auf Basophilen und Mastzellen sowie auf deren Vorläuferzellen und auf der Mastzellinie HMC-1 sowie der basophilen Leukämiezellinie KU-812 exprimiert ist. Aufgrund der DE 197 08 877 C1 war dies nicht zu erwarten, denn dort wird beschrieben, daß der Antikörper 97A6 spezifisch für Megakaryozyten ist. Neuere Forschungsergebnisse des Erfinders zeigen jedoch, daß der Antikörper 97A6 zwar einige wenige megakaryozytische Zellinien, jedoch keine nativen Megakaryozyten erkennt.

Da sich der erfindungsgemäß verwendete Antikörper gut zum Aufreinigen von Zellen eignet, betrifft die Erfindung auch eine im wesentlichen reine Population von Basophilen und/oder Mastzellen und/oder Vorläuferzellen von Basophilen und/oder Mastzellen, wobei die Zellen von einem Reagenz gebunden werden können, das spezifisch an solche Oberflächenstrukturen dieser Zellen bindet, die von dem Antikörper 97A6 erkannt werden. Darüber hinaus betrifft die Erfindung ein entsprechendes Reagenz zum Binden dieser Zellen.

Mit Hilfe des Antikörpers 97A6 ist es leicht möglich, die Oberflächenstruktur, an die der Antikörper bindet, zu isolieren und dagegen sowohl monoklonale als auch polyklonale Antikörper herzustellen.

Mittels FACS-Analyse konnte inzwischen gezeigt werden, daß es sich bei der Oberflächenstruktur um das Phosphodiesterase/nucleotid-pyrophosphatase-Ektoenzym PDNP3 handelt, das auch als NPP3 oder PD-Ibeta bezeichnet wird. Die Sequenz wurde veröffentlicht von Jin-Hua et al. in Genomics 45, 412-415 (1997).

Ein großer Vorteil der erfindungsgemäßen Verwendung eines Antikörpers besteht darin, daß damit zuverlässig die Überwachung der Zahl von Basophilen im Blut von Patienten mit chronischmyeloischer Leukämie ermöglicht wird, weil viele der in unreifer Form vorliegenden Basophilen, die bisher morphologisch nicht als solche identifizierbar waren, damit zuverlässig erkannt werden können. Somit kann die genaue Zahl von Basophilen in diesen Patienten und damit der Status ihrer Erkrankung bestimmt werden.

Weiterhin ermöglicht die erfindungsgemäße Verwendung eines Antikörpers vorteilhafterweise die Isolierung von Vorläuferzellen, aus denen je nach Kultivierungsbedingungen Mastzellen oder Basophile generiert werden können. Somit erleichtert die erfindungsgemäße Verwendung die Erforschung der Differenzierung dieser Zellen. Besonders vorteilhaft gegenüber anderen bisher bekannten Antikörpern ist dabei, daß Basophile schon in sehr frühen Entwicklungsstadien mit dem verwendeten Antikörper detektierbar sind und in der weiteren Entwicklung damit detektierbar bleiben.

Ein für die Routine-Diagnostik wesentlicher Vorteil der erfindungsgemäßen Verwendung eines Antikörpers besteht in der Möglichkeit, mit einem einzigen Antikörper beispielsweise Mastzellen aus verschiedenen Geweben und Basophile aus Blut nachweisen zu können. Bisher waren dazu verschiedene Antikörper und Antikörperkombinationen notwendig, was mit deutlichen Mehrkosten verbunden war. Basophile können in Blutproben auch histochemisch gefärbt werden, beispielsweise mittels May-Grünwald-Giemsa. Zur Identifizierung und Auszählung der Basophilen ist dann erfahrenes Personal notwendig, und subjektive Schwankungen der Ergebnisse sind möglich. Unreife Basophile sind auf diese Weise nicht zu identifizieren. Dagegen ermöglicht die erfindungsgemäße Verwendung von Antikörpern die Analyse mittels objektiv auswertbarer Standardverfahren, wie ELISA oder FACS, die auch von weniger erfahrenem Personal durchgeführt werden können und einen wesentlich größeren Probendurchsatz ermöglichen als die subjektive Auswertung von Färbungen.

Weiterhin haben die Erfinder der vorliegenden Erfindung erkannt, daß die Aktivierung von Basophilen, beispielsweise im Zusammenhang mit einer allergischen Reaktion, zu einer verstärkten Präsentation der durch den erfindungsgemäß verwendeten Antikörper erkannten Oberflächenstruktur führt. Daher eignet sich der Nachweis und/oder die Quantifizierung dieser Oberflächenstruktur auf Basophilen gut zur Untersuchung der Aktivierung von Basophilen.

In einer bevorzugten Ausgestaltung ist der verwendete Antikörper ein monoklonaler Antikörper.

Die Verwendung eines monoklonalen Antikörpers hat den Vorteil, daß der Antikörper standardisiert reproduzierbar ist und somit potentiell in unbegrenzten Mengen hergestellt werden kann. Darüber hinaus sind die Bindungseigenschaften eines monoklonalen Antikörpers stets konstant, so daß auch jede Verwendung eines monoklonalen Antikörpers standardisierbar ist.

In einer weiteren bevorzugten Ausgestaltung wechselwirkt der erfindungsgemäß verwendete Antikörper im wesentlichen nicht mit Immunglobulinen der Klasse IgE.

Die Verwendung eines solchen Antikörpers weist bei Untersuchungen im Zusammenhang mit der Aktivierung von Basophilen nämlich den unerwarteten Vorteil auf, daß er die Basophilen nicht durch Vernetzung von zellgebundenen IgE-Immunglobulinen selbst aktiviert und die Unterschungsergebnisse somit nicht beeinflußt.

In einer weiteren Ausführungsform der vorliegenden Erfindung wird der Antikörper 97A6 selbst zur Detektion und/oder Isolierung verwendet.

Die Verwendung dieses Antikörpers hat den Vorteil, daß dieser Antikörper bereits gut charakterisiert ist und in großen Mengen zur Verfügung steht.

Die Erfindung betrifft ferner die erfindungsgemäße Verwendung eines Antikörpers im Zusammenhang mit der Analyse der Hämatopoese.

Wie zuvor bereits erwähnt, eröffnet die erfindungsgemäße Verwendung die Möglichkeit, Vorläuferzellen von Basophilen und Mastzellen zu isolieren und so die Hämatopoese in bezug auf diese Zellen zu analysieren. Diese Analyse ist jedoch nicht nur von wissenschaftlichem Interesse, sondern kann auch bei der Untersuchung von Blutbildungsstörungen in der klinischen Diagnostik eingesetzt werden.

In einer bevorzugten Ausgestaltung erfolgt die erfindungsgemäße Verwendung eines Antikörpers im Zusammenhang mit der Analyse von Patientenproben, insbesondere von Gewebebiopsien, Knochenmarkbiopsien und/oder Blutproben. Dabei dient die erfindungsgemäße Verwendung bei Gewebebiopsien zum Nachweis von Mastzellen, bei Knochenmarkbiopsien zum Nachweis von Vorläuferzellen von Mastzellen und/oder Basophilen und bei Blutproben zum Nachweis von reifen und unreifen Basophilen.

Wie bereits erwähnt, sind somit mit einem einzigen Antikörper, je nach Ausgangsmaterial, verschiedene Analysen möglich, die bisher nur mit Antikörperkombinationen oder überhaupt nicht durchführbar waren.

Die erfindungsgemäße Verwendung eines Antikörpers betrifft ferner die diagnostische Einordnung von Tumoren, insbesondere von Leukämien.

Die Diagnose und Klassifizierung von Leukämien erfolgt anhand von Knochenmarkbiopsien oder Blutproben. So wird beispielsweise bei einer Ausprägungsform der Leukämie, der CML, anhand von Knochenmarkuntersuchungen der Status aller myeloischen Zellen analysiert. Der erfindungsgemäße Antikörper hat dabei den Vorteil, Informationen über den Anteil von Vorläuferzellen für Basophile und Mastzellen zu liefern. Bei einer Blutuntersuchung ermöglicht die erfindungsgemäße Verwendung - wie bereits erwähnt - die Detektion von Basophilen, insbesondere unreifen Basophilen, die bisher nicht erkannt werden konnten. Da die Zahl von Basophilen aber ein kritischer Parameter bei der Progression der CML ist, bietet die erfindungsgemäße Verwendung den Vorteil, eine zuverlässigere diagnostische Aussage über das Stadium der CML zu ermöglichen, als dies bisher möglich war.

Bei einer weiteren bevorzugten Ausgestaltung der Erfindung ist der verwendete Antikörper mit einem Marker, insbesondere mit einem Fluoreszenz-Marker, verbunden.

Hierbei ist vorteilhaft, daß der Antikörper dann mit hoher Sensitivität nachgewiesen werden kann, weshalb nur kleine Mengen des Antikörpers zur Diagnose eingesetzt werden müssen. Darüber hinaus ist die Verwendung eines solchen Antikörpers bei einem ELISA oder bei der Anwendung der Durchflußzytometrie möglich.

Bei einer weiteren erfindungsgemäßen Ausgestaltung erfolgt der Nachweis gebundener Antikörper durch ein übliches immunologisches Nachweisverfahren, insbesondere ELISA oder FACS-Analyse.

Diese Verwendung hat den Vorteil, daß sie eine hochspezifische, sensitive, schnelle und in automatisierter Form durchführbare Bestimmung von Zellen in Patientenproben erlaubt.

Die Erfindung betrifft ferner die erfindungsgemäße Verwendung eines Antikörpers zum Nachweisen und/oder Quantifizieren von aktivierten Basophilen.

Eine solche Verwendung hat den Vorteil, daß dadurch die Ursachen einer Aktivierung von Basophilen relativ einfach untersucht werden können, weil aktivierte Basophile gegenüber nicht aktivierten eine größere Zahl von erfindungsgemäß verwendeten Antikörpern binden. So können Basophile beispielsweise mit potentiell aktivierenden Agenzien und erfindungsgemäß verwendeten Antikörpern inkubiert werden. Anschließend können aktivierte Basophile von nicht aktivierten durch die erhöhte Expression des 97A6-Antigens unterschieden werden. Diese erhöhte Expression kann im Durchflußzytometer quantifiziert werden.

Die erfindungsgemäße Verwendung eines Antikörpers betrifft ferner die Bestimmung des Ausmaßes der Aktivierung von Basophilen.

Durch die Bestimmung des Ausmaßes der Antikörperbindung an einzelne Basophile kann auch das Ausmaß der Antigenexpression und somit der Aktivierung dieser Zellen bestimmt werden. Somit eröffnet diese erfindungsgemäße Verwendung die vorteilhafte Möglichkeit Agenzien, die Basophile aktivieren, daraufhin zu untersuchen, in welchem Ausmaß sie zur Aktivierung dieser Zellen beitragen. Somit können Agenzien, beispielsweise Allergene, nach ihrer Fähigkeit eingestuft werden, Basophile zu aktivieren. Auf diese Art und Weise kann beispielsweise eine Abschätzung des allergieauslösenden Potentials eines Agens erfolgen.

Die Erfindung betrifft ferner ein Verfahren zur Untersuchung von Allergien, mit den Schritten:
- Inkubieren einer Blutprobe mit einem Agens, das im Verdacht steht, eine allergische Reaktion auszulösen,
- Inkubieren dieser Blutprobe mit einem in den Ansprüchen 1 bis 4 verwendeten Antikörper,
- Quantifizieren der an Zellen gebundenen Antikörper.

Dieses erfindungsgemäße Verfahren eröffnet die Möglichkeit, einen Allergietest durchzuführen, ohne daß der zu untersuchenden Person ein lästiger und unangenehmer Hauttest zugemutet werden muß. Für das erfindungsgemäße Verfahren ist es lediglich notwendig, mit einigen Millilitern Blut von der zu untersuchenden Person in vitro einen Test mit verschiedenen Allergenen durchzuführen, wobei 15 Minuten nach Inkubieren mit einem Agens der erfindungsgemäß verwendete Antikörper inkubiert werden kann. Durch die Quantifizierung der von den Basophilen gebundenen Antikörper kann dabei nicht nur eine Aussage darüber gemacht werden, ob ein Agens eine allergische Reaktion auslösen kann, sondern auch darüber, wie stark diese allergische Reaktion ist.

Die Erfindung betrifft ferner ein Verfahren zur Bereitstellung von hämatopoetischen Vorläuferzellen, die in Mastzellen oder Basophile differenzieren können, mit den Schritten:
- Isolieren und Bereitstellen von Knochenmarkzellen aus einem Organismus,
- Inkubieren dieser Knochenmarkzellen mit einem erfindungsgemäß verwendeten Antikörper,
- Isolieren der antikörpergebundenen Zellen mit üblichen Verfahren, insbesondere FACS und MACS (magnetic cell sorting).

Die Bereitstellung dieser hämatopoetischen Vorläuferzellen ermöglicht die Untersuchung der Entwicklung von Mastzellen und/oder Basophilen. Dies kann zu Forschungszwecken und im Falle einer gestörten Hämatopoese auch zu diagnostischen Zwecken erfolgen. Darüber hinaus können aus Vorläuferzellen *in vitro* Zellen gezüchtet werden, die später wieder in den Spender der Vorläuferzellen eingebracht werden können, ohne immunologische Probleme zu verursachen. Dies kann beispielsweise bei Patienten mit einer gestörten Hämatopoese sinnvoll sein.

Bei Basophilen oder Mastzellen handelt es sich nicht um Zelllinien, sondern um Primärzellen, die erst in Kenntnis der Erfindung identifiziert und aufgereinigt werden können.

Der Vorteil einer solchen Population besteht beispielsweise darin, daß damit sehr spezifisch die Eigenschaften der Zellen erforscht werden können, ohne daß die Ergebnisse durch andere Zellen beeinflußt werden. Dies ist beispielsweise dann wichtig, wenn erforscht werden soll, welche Substanzen von bestimmten Zellen unter bestimmten Bedingungen produziert und abgegeben werden, weil diese Substanzen in der Regel nur von einer großen Population von Zellen in so ausreichenden Mengen hergestellt werden, daß damit eine Analyse bzw. eine Identifizierung dieser Substanzen möglich ist. Würde die Population unterschiedliche Zelltypen enthalten, könnte aus dem Nachweis einer bestimmten Substanz nicht darauf geschlossen werden, welche Zellen diese Substanz produzieren.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

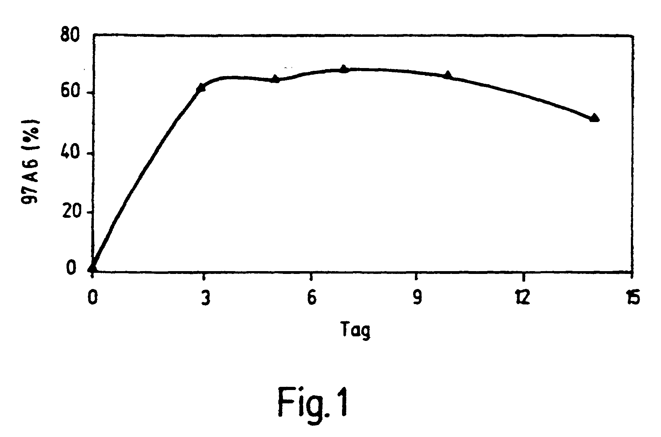

Weitere Vorteile ergeben sich aus den folgenden Ausführungsbeispielen und im Zusammenhang mit der Zeichnung, in der
- Fig. 1: ein Diagramm zeigt, in dem der prozentuale Anteil 97A6-positiver CD34⁺-Zellen aus peripherem Blut, die 0, 3, 5, 7, 10 und 14 Tage lang in Gegenwart von 100 ng/ml IL-3 *in vitro* kultiviert wurden, dargestellt ist;

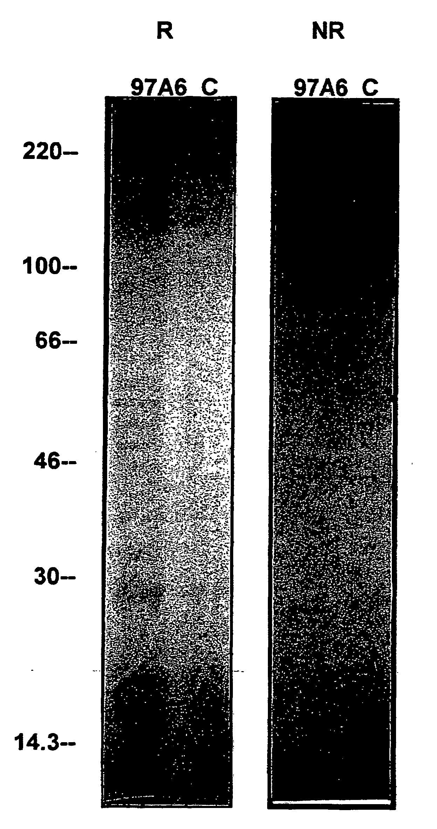

- Fig. 2: eine Immunpräzipitation von 10⁷, mit ¹²⁵I oberflächenmarkierten und anschließend lysierten KU-812-Zellen mit dem Antikörper 97A6 (Spur 97A6) sowie eine Kontrollpräzipitation ohne den Antikörper 97A6 (Spur C) zeigt, die auf einem 5-15 % SDS-Polyacrylamidgel unter reduzierenden (R) und unter nicht-reduzierenden (NR) Bedingungen elektrophoretisch aufgetrennt und autoradiographisch sichtbar gemacht wurden; Molekulargewicht-Standards sind links angezeigt;

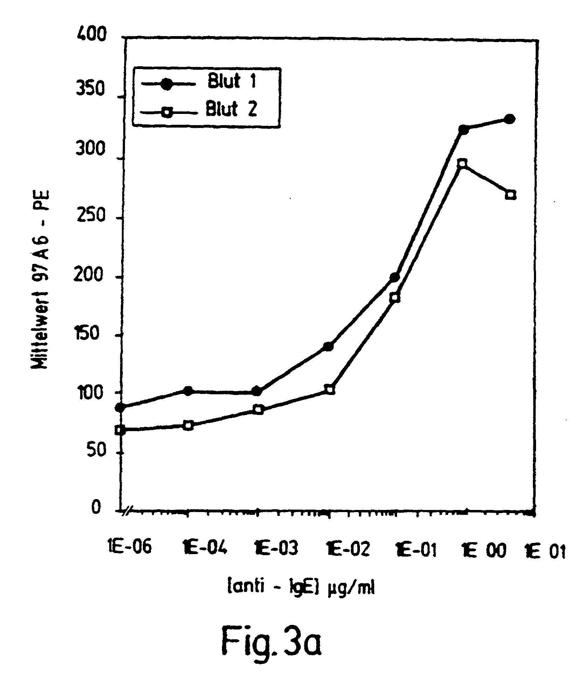

- Fig. 3a: ein Diagramm zeigt, in dem die durchschnittlich an Basophile gebundene Menge des mit Phycoerythrin markierten Antikörpers 97A6 (97A6-PE) in willkürlichen Fluoreszenzintensitätseinheiten (Mittelwert 97A6-PE) dargestellt ist, wobei die Basophilen zuvor mit den in dem Diagramm angegebenen Anti-IgE-Antikörperkonzentrationen inkubiert worden sind und aus dem Blut eines Spenders stammen, der gegen Akarinen allergisch ist (Blut 1) oder aus dem Blut eines Spenders stammen, der nicht allergisch ist (Blut 2); und

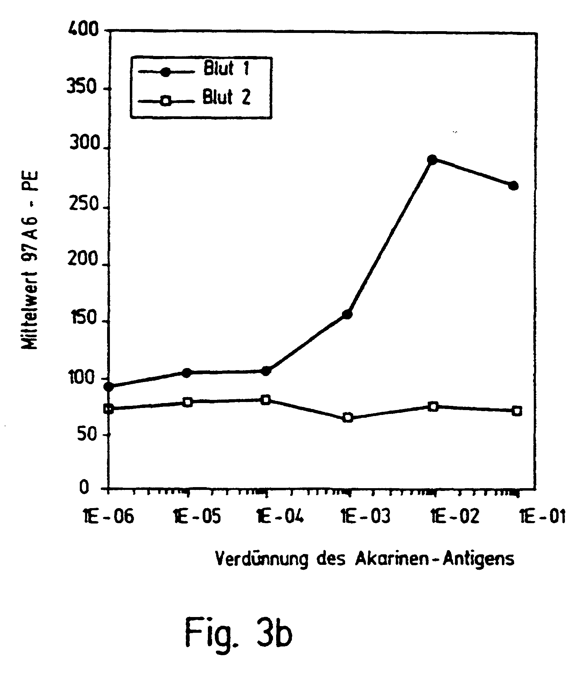

- Fig. 3b: ein Diagramm zeigt, in dem die durchschnittlich an Basophile gebundene Menge von 97A6-PE in willkürlichen Fluoreszenzintensitätseinheiten (Mittelwert 97A6-PE) dargestellt ist, wobei die Basophilen zuvor mit den in dem Diagramm angegebenen Verdünnungen des Akarinen-Antigens inkubiert worden sind und aus dem Blut eines Spenders stammen, der gegen Akarinen allergisch ist (Blut 1) oder aus dem Blut eines Spenders stammen, der nicht allergisch ist (Blut 2).

### Beispiel 1:

Isolierung und Nachweis von Basophilen aus peripherem Blut von Normalpersonen und Leukämie-Patienten.

Es ist bekannt, daß die Zahl der Basophilen im peripheren Blut von Patienten mit CML beim Übergang von der chronischen in die beschleunigte Phase ansteigt.

Um die Spezifität des Antikörpers 97A6 für Basophile zu testen, wurden Mononukleäre Leukozyten über Ficoll-Hypaque von anderen Bestandteilen von Buffy Coats aus peripherem Blut von Normalpersonen oder Patienten mit CML in der Akzelerationsphase getrennt. Die Interphase-Zellen wurden anschließend mit dem Biotin-markierten Antikörper 97A6 inkubiert, dreimal gewaschen und mit Anti-IgG-MACSbeads (Miltenyi, Bergisch Gladbach, Deutschland) inkubiert. 97A6-positive Zellen wurden dann durch MACS isoliert und anschließend zur Kontrolle der Präparation mit Phycoerythrin (PE) -markiertem Streptavidin und einem PE-markierten 97A6-Antikörper markiert und in einem Durchflußzytometer analysiert.

Die Reinheit der 97A6-positiven Zellen war 99 %.

Zur morphologischen Analyse wurden diese Zellen mit May-Grünwald-Giemsa gefärbt. Dabei zeigte sich bei Normalpersonen auch morphologisch eine reine Basophilen-Population, während nur 60 bis 80 % der 97A6-positiven Zellen von CML-Patienten in der Akzelerationsphase morphologisch als Basophile identifizierbar waren. Die restlichen, morphologisch unidentifizierbaren 97A6-positiven Zellen erwiesen sich als unreife Vorläuferzellen von Basophilen.

Dies zeigt, daß durch den Antikörper 97A6 ein größerer Anteil der Basophilen erkannt wird als durch das bisher verwendete Färbeverfahren.

### Beispiel 2:

Detektion von Gewebe-Mastzellen mit dem Antikörper 97A6.

Zungen-, Vorhaut- oder Gebärmuttergewebe wurde zerkleinert, in einem Puffer mit 200 mg/l KCl, 50 mg/l NaH₂PO₄ x H₂O, 8 g/l NaCl und 1 g/l Glukose gewaschen und anschließend 90 bis 180 min bei 37 °C mit 2 mg/ml Kollagenase Typ II (Sebak, Suben, Österreich) inkubiert. Danach wurden die dispergierten Zellen abzentrifugiert, gewaschen und 30 min mit humanem AB-Serum inkubiert. Nach erneutem Waschen wurden die Zellen 30 min bei 4 °C mit dem Antikörper 97A6 inkubiert, gewaschen und dann 30 min bei 4 °C mit einem Fluorescein-markierten Ziegen F(ab')₂ IgG-Anti-Maus-Antikörper inkubiert. Die Zellen wurden 1 min in 0,025 % Glutaraldehyd fixiert, gewaschen und 8-12 min mit 0,0125 % Toluidin-Blau inkubiert. Nach dem Waschen wurden Toluidin-Blau gefärbte Mastzellen mittels eines Fluoreszenz-Mikroskopes (Olympus, Wien, Österreich) im Hellfeld aufgrund ihrer Morphologie identifiziert und anschließend im Fluoreszenzlicht untersucht.

Es zeigte sich, daß ausschließlich Mastzellen aus allen Geweben, nicht jedoch andere Gewebezellen mit dem Antikörper 97A6 markiert waren. Darüber hinaus zeigten Mastzellen eine deutlich geringere Fluoreszenz-Färbung als gleichartig gefärbte Basophile.

### Beispiel 3:

### Isolierung und Kultivierung von Vorläuferzellen

Zellen aus Knochenmarkproben wurden auf Ficoll-Hypaque, das eine Dichte von 1,077 g/cm³ aufwies, zentrifugiert. Die Zellen der Interphase wurden isoliert, und Zellen, die für CD34, einem Marker für hämatopoetische Vorläuferzellen, positiv waren, wurden mittels MACS isoliert. Diese Zellen wurden mit einem FITCmarkierten Antikörper gegen CD34 und dem PE-markierten Antikörper 97A6 inkubiert. Doppelt positive Zellen wurden mittels FACS selektioniert und in einem semi-soliden Fertigmedium mit 0,9 % Methylzellulose und einer Mischung von Wachstumsfaktoren einschließlich IL-3, einem Differenzierungsfaktor für Basophile und Eosinophile (CellSystems, Remagen, Deutschland), ausgesät.

Nach 16-tägiger Inkubation bei 37 °C in einer befeuchteten Atmosphäre (5 % CO₂) wurden die entstandenen Kolonien ausgezählt, mit einer Pasteur-Pipette auf Objektträger übertragen und zur morphologischen Untersuchung mit May-Grünwald-Giemsa gefärbt. Dabei zeigten sich neben den überwiegenden reinen Basophilen-Kolonien auch gemischte Eosinophile-Basophile-, Basophile-Makrophagen- und multipotente Basophile-Eosinophile-Makrophagen- und/oder Neutrophile-Kolonien.

Dies zeigt, daß 97A6-positive Vorläuferzellen entweder auf die Entwicklung eingeschränkte, bipotente oder multipotente Vorläuferzellen von verschiedenen myeloischen Zellen einschließlich Basophilen sind.

### Beispiel 4:

### 97A6-Antigenexpression während der Basophilen-Entwicklung

IL-3 ist ein Stimulator für Wachstum und Differenzierung von Basophilen und deren Vorläuferzellen. Zur Erzeugung von Basophilen aus Vorläuferzellen wurden CD34-positive Zellen mittels MACS aus peripherem Blut isoliert und in Serum-freiem IMDM-Medium mit 700 µg/ml Holo-Transferrin, 40 µg/ml niedermolekularen humanen Proteinen und 10 µg/ml Insulin (Sigma, München, Deutschland) kultiviert und mit 100 ng/ml IL-3 (Behring-Werke, Marburg, Deutschland) 0, 3, 5, 7, 10 und 14 Tage lang stimuliert. Zur Untersuchung der Expression des 97A6-Antigens wurden die Zellen danach wie bei Beispiel 1 beschrieben mit Hilfe des Antikörpers 97A6 markiert. Der Anteil 97A6-positiver Zellen wurde durchflußzytometrisch bestimmt.

Dabei zeigte sich ein signifikanter Anstieg des Anteils 97A6-positiver Zellen von 1 ± 0,5 % am Tag Null der Kultivierung auf über 60 % am dritten Tag der Kultivierung. Anschließend blieb dieser Anteil etwa konstant und fiel erst gegen Ende der 14-tägigen Kultivierung geringfügig ab (Fig. 1).

Bei der morphologischen Beurteilung der 97A6-positiven Zellen nach einer May-Grünwald-Giemsa-Färbung zeigten sich überwiegend Basophile. Der rasche Anstieg des Anteils 97A6-positiver Zellen zeigt, daß das 97A6-Antigen schon in frühen Stadien der Basophilen-Entwicklung exprimiert wird. Dies wird auch durch den in Beispiel 3 beschriebenen Versuch demonstriert, bei dem mit Hilfe des 97A6-Antigens Vorläuferzellen von Basophilen isoliert wurden. Auch in Beispiel 1 wurde dieses Ergebnis bestätigt, indem aus peripherem Blut von Patienten mit CML in der beschleunigten Phase 97A6-positive Zellen als unreife basophile Vorläuferzellen identifiziert werden konnten.

### Beispiel 5:

### Immunpräzipitation von Zelloberflächenmolekülen mit dem Antikörper 97A6

Zellen der basophilen Vorläuferzellinie KU-812 wurden nach dem Lactoperoxidase-Verfahren mit ¹²⁵I oberflächenmarkiert und anschließend mit NP-40 lysiert. Die Immunpräzipitation wurde mit vorgebildeten Komplexen von 97A6-Antikörpern durchgeführt. Die 97A6-Antikörper waren an Sepharose-Beads immobilisiert, die mit sekundären Antikörpern konjugiert waren. Als Kontrolle wurde eine Immunpräzipitation mit Beads ohne den 97A6-Antikörper durchgeführt. Nach dem Kochen der Proben in SDS unter reduzierenden Bedingungen in Gegenwart von 2-Mercaptoethanol und unter nicht-reduzierenden Bedingungen wurden die apparenten Molekulargewichte durch gelelektrophoretische Auftrennung auf 5-15-prozentigen Polyacrylamidgelen (Laemmli, UK, Nature 227 (1970), 680) unter Verwendung von ¹⁴C-methylierten Markerproteinen nach Autoradiographie bestimmt.

Das Ergebnis dieser Immunpräzipitation ist in Fig. 2 gezeigt. Mit dem Antikörper 97A6 können Proteine mit einem apparenten Molekulargewicht von ca. 270 kD und ca. 150 kD unter reduzierenden Bedingungen und von ca. 270 kD unter nicht-reduzierenden Bedingungen präzipitiert werden. Bei dem Protein-Komplex handelt es sich um eine unbekannte Oberflächenstruktur, die auf Basophilen, Mastzellen und deren Vorläuferzellen vorkommt.

Um die molekulare Struktur des detektierten Antigens zu identifizieren, wurden KU-812 Lysate auf einer 97A6 Antikörper-Säule gereinigt. Das eluierte Protein wurde durch SDS-PAGE separiert, und die sich ergebenden Banden wurden einer kommerziellen Aminosäuresequenzierung unterzogen. Ein Sequenzvergleich mit Datenbanksequenzen ergab eine 100-prozentige Identität mit dem kürzlich klonierten PDNP3/NPP3 Phosphodiesterase/nucleotidpyrophosphatase-Ektoenzym.

### Beispiel 6:

### Koexpression von CD-Antigenen auf 97A6-positiven Zellen

Knochenmarkzellen **(BM),** Zellen des peripheren Blutes **(PB),** Zellen des peripheren Blutes von CML-Patienten **(PB CML)** und CD34-positive Zellen des peripheren Blutes, die mit 100 ng/ml IL-3 stimuliert wurden und nach drei Tagen **(d3 PB (+IL-3))** bzw. nach 14 Tagen **(d14 PB (+IL-3))** geerntet wurden, wurden mit 97A6-PE, FITC-konjugierten Antikörpern der angegebenen Spezifität und mit Anti-CD34-PerCP-Antikörpern (HPCA-2-PerCP) (Becton Dickinson, Heidelberg, Deutschland) gefärbt. Die FITC-konjugierten Antikörper gegen CD10 (W8E7), CD26 (L272), CD44 (L178), CD71 (L01.1) und HLA-DR (L243), (Becton Dickinson), CD13 (SJ1D1), CD16 (3G8), CD33 (D3HL60.251), CD38 (T16), (Immunotech, Krefeld, Deutschland), CD117 (9B9), (Hölzel Diagnostics, Köln, Deutschland), IgE (polyklonal), (BioSource, Ratingen, Deutschland), CD55 (1A10) und CD59 (P282H19), (PharMingen, Hamburg, Deutschland), CD123 (7G3), (PharMingen), CD164 (103B2/9E10), (Zannettino A.C.B. et al., Blood 92 (1998), 2613), CD81 (JS-64), (Immunotech) und CDw17 (MEM74), (BioSource) wurden jeweils zusammen mit den Zellen, dem Antikörper 97A6-PE und Anti-CD34-PerCP 30 Minuten auf Eis inkubiert, zweimal gewaschen und mittels eines Durchflußzytometers analysiert. Die Ergebnisse sind in Tabelle 1 aufgeführt. "+/-" zeigen positive Subpopulationen an, "n.d." bedeutet, daß das entsprechende Antigen nicht nachgewiesen wurde.

**Tabelle 1:**

| Koexpression von CD-Antigenen auf 97A6-positiven Zellen | | | | | | | |
|---|---|---|---|---|---|---|---|
| | **PB CD34**^{**-**} | **PB CD34**^{**+**} | **BM CD34**^{**-**} | **BM CD34**^{**+**} | **PB CML** | **d3 PB (+ IL-3)** | **d14 PB (+ IL-3)** |
| CD10 | - | n.d. | - | - | - | n.d. | - |
| CD13 | + | - | +/- | +/- | +/- | - | +/- |
| CD16 | - | n.d. | n.d. | n.d. | +/- | n.d. | - |
| CD17 | + | - | +/- | +/- | +/- | +/- | - |
| CD26 | + | - | + | +/- | + | +/- | +/- |
| CD33 | +/- | + | + | + | + | + | |
| CD38 | + | + | + | + | + | + | +/- |
| CD44 | + | + | + | + | + | + | +/- |
| CD55 | + | + | + | + | + | + | + |
| CD59 | + | + | + | + | + | + | + |
| CD71 | - | + | +/- | + | - | + | +/- |
| CD81 | +/- | n.d. | + | + | + | + | + |
| CD117 | - | + | - | + | - | + | n.d. |
| CD123 | + | + | + | + | + | n.d. | |
| CD164 | + | + | + | + | + | - | +/- |
| a-IgE | + | +/- | + | + | - | - | +/- |
| HLA-DR | - | +/- | +/- | + | - | + | +/- |

Diese Studie zeigt, daß reife Basophile (CD34-negativ) des peripheren Blutes negativ für CD117, einem Marker für Mastzellen, und negativ für CD71, einem Marker für proliferierende Zellen, sind, jedoch CD17 und eine hohe Dichte der IL-3-Rezeptor-Alpha-Kette (CD123), einem Marker für Basophile, Eosinophile und Monozyten, exprimieren. CD34-negative Knochenmarkzellen scheinen CD13, CD17, CD71 und HLA-DR heterogener zu exprimieren als Basophile des peripheren Blutes. Eine ähnliche Expression wird auf Basophilen des peripheren Blutes von CML-Patienten in der Akzelerationsphase gefunden. Im Gegensatz zu den entsprechenden Knochemarkzellen sind alle CD34⁺97A6⁺-Basophilenvorläufer des peripheren Blutes negativ für CD13, CD17 und CD26. Dies zeigt, daß die Vorläufer aus dem peripheren Blut etwas unreifer sind. Beide Vorläufer sind jedoch positiv für CD117 und CD71, die auf reifen Basophilen nicht vorkommen. Anders als reife Basophile aus dem peripheren Blut exprimieren kultivierte 97A6⁺-Basophile, die aus CD34-positiven Zellen des peripheren Blutes durch Stimulation mit IL-3 erhalten wurden, nur wenig oder kein CD38, aber eine hohe Dichte von HLA-DR-Molekülen.

Diese Daten zeigen, daß das 97A6-Antigen kontinuierlich in allen Stadien der Basophilenreifung exprimiert wird, während die Expression von CD-Antigenen auf Basophilen von unterschiedlichem Reifungsgrad und aus unterschiedlichen Quellen deutlich schwankt.

### Beispiel 7:

Nachweis der Aktivierung von Basophilen mittels des Antiköpers 97A6.

In dieser in den Fig. 3a und 3b dargestellten Studie wurde je eine Blutprobe eines Spenders, der gegen Akarinen allergisch ist (Blut 1), und eine Blutprobe eines Spenders, der nicht allergisch ist (Blut 2), zur Gerinnungsinhibition mit Heparin behandelt. Anschließend wurden jeweils 100 µl Blut jeder Probe entweder mit einem Anti-IgE-Antikörper (Klon E124-2-8/Dε2, Maus IgG 1, Immunotech) in den in Fig. 3a angegebenen Endkonzentrationen oder mit Akarinen-Antigen (Stallergenes, Anthony, Frankreich) in den in Fig. 3b angegebenen Verdünnungen 15 Minuten bei 37 °C inkubiert. Nach dem Waschen einer jeden Probe mit PBS/20 mM EDTA und anschließendem Abzentrifugieren der Zellen wurden die Zell-Pellets in 100 µl PBS/BSA resuspendiert. Zu jeder Probe wurden 20 µl des Antikörpers 97A6-PE in einer Konzentration von 25 µg/ml zugesetzt. Nach 15-minütiger Inkubation bei Raumtemperatur im Dunkeln wurden die Zellen gewaschen und die roten Blutzellen mittels des IOTest-Lysing-Reagenz (Immunotech Nr. 486) lysiert. Nach erneutem Waschen und Resuspendieren der Zellen in PBS/BSA wurde die Fluoreszenz in einem Durchflußzytometer gemessen.

Dabei entsprach die Fluoreszenz-Färbung bei den niedrigsten Konzentrationen der Aktivatoren Anti-IgE und Akarinen-Antigen derjenigen Fluoreszenz-Färbung ohne diese Aktivatoren.

Das in der Fig. 3a dargestellte Ergebnis zeigt, daß der für Basophile bekannte Aktivierungsmechanismus, bei dem IgE, das auf der Zelloberfläche von Basophilen gebunden ist, durch Antikörper vernetzt wird, im Rahmen der Aktivierung zu einer sehr schnellen Präsentation derjenigen Oberflächenstruktur auf den Basophilen führt, die vom Antikörper 97A6 erkannt wird.

Das in Fig. 3b dargestellte Ergebnis zeigt, daß nur diejenigen Basophilen, die von dem Spender stammen, der gegen Akarinen allergisch ist, auf die Inkubation mit Akarinen-Antigen mit einer verstärkten Präsentation derjenigen Oberflächenstruktur reagieren, die von dem Antikörper 97A6 erkannt wird. Die Basophilen aus dem Blut des nicht allergischen Spenders zeigen dagegen nach der Inkubation mit dem Akarinen-Antigen keine verstärkte Präsentation dieser Oberflächenstruktur.

Der dahinterstehende Mechanismus besteht höchstwahrscheinlich darin, daß die auf den Basophilen des allergischen Spenders gebundenen IgE-Immunglobuline das Akarinen-Antigen erkennen können und durch die Inkubation mit diesem Antigen vernetzt werden, so daß es zu einer Aktivierung der Basophilen kommt und die Basophilen daraufhin ebenso wie bei der Aktivierung durch Anti-IgE-Antikörper mit einer verstärkten Präsentation der Oberflächenstruktur reagieren, die von dem Antikörper 97A6 erkannt wird. Das Ausbleiben einer Reaktion bei den Basophilen des nicht allergischen Spenders zeigt außerdem, daß der Antikörper 97A6 selbst zu keiner Aktivierung der Basophilen durch IgE-Bindung führt.

Bemerkenswert ist dabei, daß für diese Präsentation eine nur 15-minütige Inkubation der Zellen bei 37 °C mit dem Antigen bzw. den Anti-IgE-Antikörpern ausreicht.

Dieses Testverfahren eröffnet die Möglichkeit, mit einer Blutprobe von wenigen Millilitern einen Allergietest durchzuführen, bei dem verschiedenste Allergene in kurzer Zeit daraufhin getestet werden können, ob sie die Basophilen des Spenders aktivieren können.

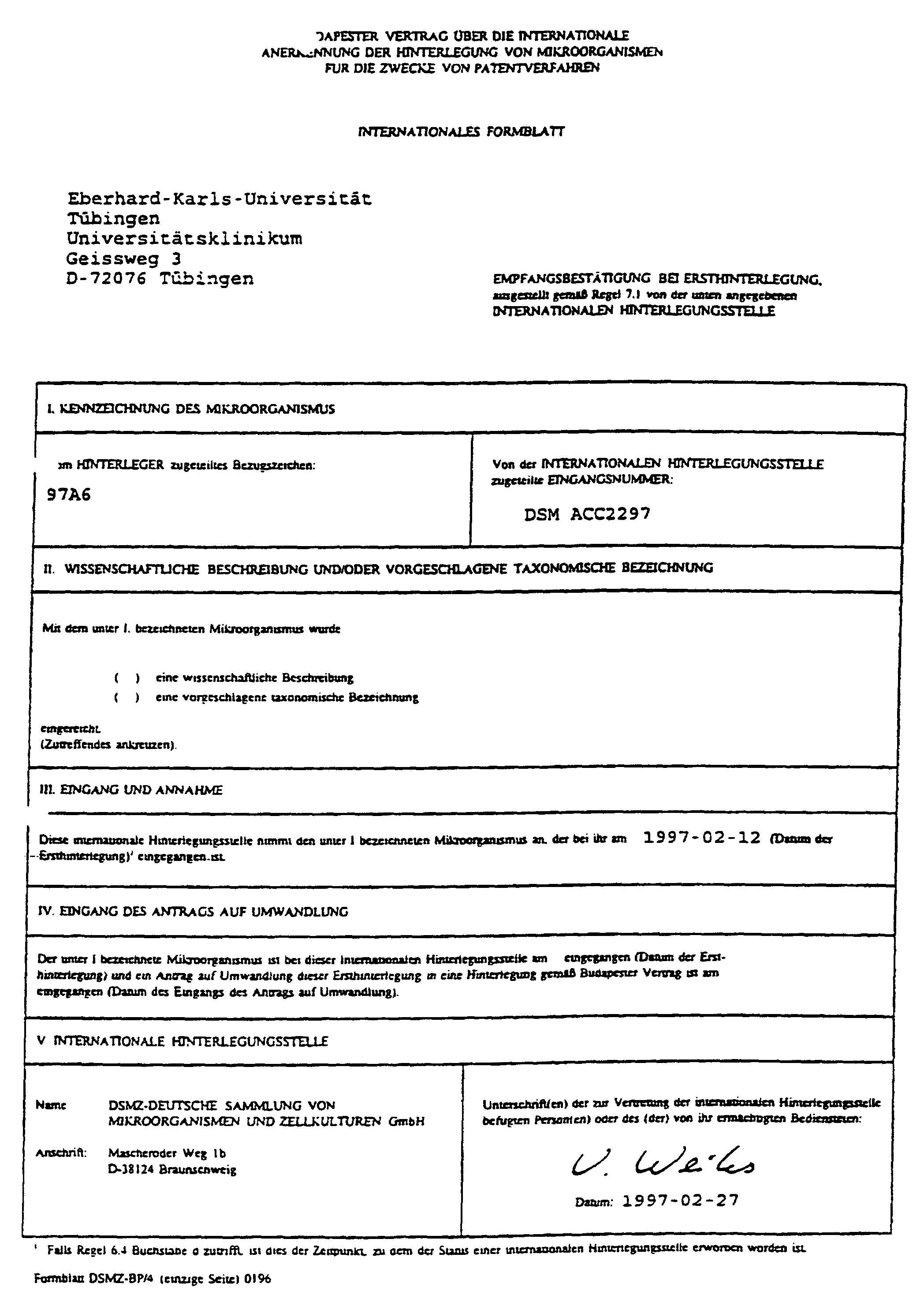

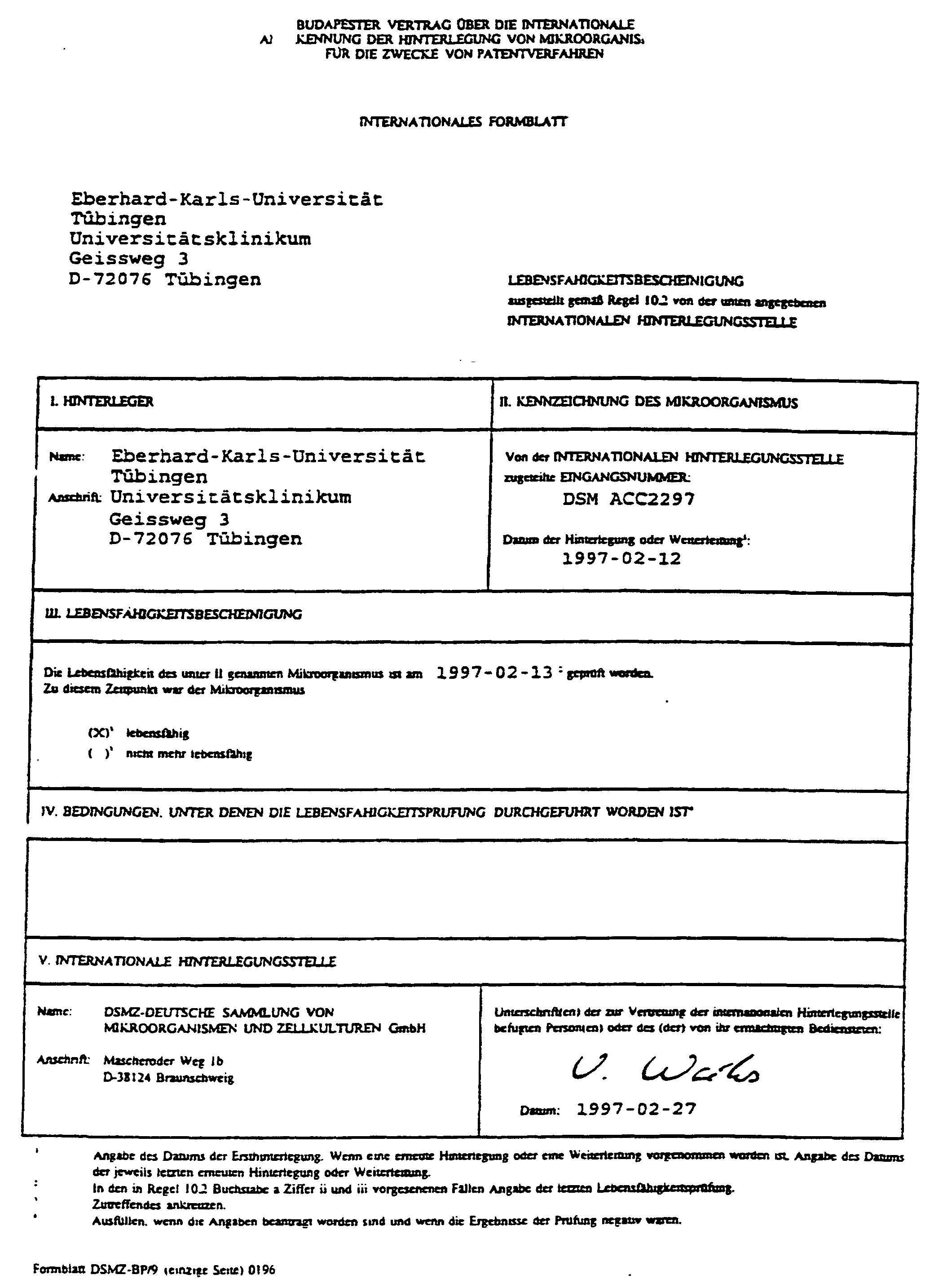

## Patentansprüche

1. Verwendung eines Antikörpers zur Detektion und/oder Quantifizierung und/oder Isolierung von Basophilen und/oder Mastzellen und/oder den Vorläuferzellen von Basophilen und/oder Mastzellen und/oder einer Oberflächenstruktur dieser Zellen, **dadurch gekennzeichnet, daß** eine Bindung des Antikörpers an die Oberflächenstruktur PDNP3 (Phosphodiesterase/nucleotid-pyrophosphatase-Ektoenzym) der Zellen erfolgt, an die der Antikörper mit der Bezeichnung 97A6 binden kann, der von Hybridomzellen produziert und freigesetzt wird, die am 12.02.1997 unter der Nummer DSM ACC 2297 bei der Deutschen Sammlung von Mikroorganismen und Zellkulturen GmbH, DSMZ, gemäß dem Budapester Vertrag hinterlegt worden sind.

2. Verwendung nach Anspruch 1, **dadurch gekennzeichnet, daß** der verwendete Antikörper ein monoklonaler Antikörper ist.

3. Verwendung nach Anspruch 1 oder Anspruch 2, **dadurch gekennzeichnet, daß** der Antikörper im wesentlichen nicht mit Immunglobulinen der Klasse IgE wechselwirkt.

4. Verwendung nach einem der Ansprüche 1 bis 3, **dadurch gekennzeichnet, daß** der Antikörper 97A6 selbst zur Detektion und/oder Isolierung verwendet wird.

5. Verwendung nach einem der Ansprüche 1 bis 4 im Zusammenhang mit der Analyse der Hämatopoese.

6. Verwendung nach einem der Ansprüche 1 bis 5 im Zusammenhang mit der Analyse von Patientenproben, insbesondere von Gewebebiopsien, Knochenmarkbiopsien und/oder Blutproben.

7. Verwendung nach einem der Ansprüche 1 bis 6 im Zusammenhang mit der diagnostischen Einordnung von Tumoren, insbesondere von Leukämien.

8. Verwendung nach einem der Ansprüche 1 bis 7, **dadurch gekennzeichnet, daß** der Antikörper mit einem Marker, insbesondere mit einem Fluoreszenz-Marker, verbunden ist.

9. Verwendung nach einem der Ansprüche 1 bis 8, **dadurch gekennzeichnet, daß** der Nachweis gebundener Antikörper durch ein übliches immunologisches Nachweisverfahren, insbesondere ELISA oder FACS-Analyse, erfolgt.

10. Verwendung nach einem der Ansprüche 1 bis 9 zum Nachweisen und/oder Quantifizieren von aktivierten Basophilen.

11. Verwendung nach einem der Ansprüche 1 bis 10 zur Bestimmung des Ausmaßes der Aktivierung von Basophilen.

12. Verfahren zur Untersuchung von Allergien, mit den Schritten:
- Inkubieren einer Blutprobe mit einem Agens, das im Verdacht steht, eine allergische Reaktion auszulösen,
- Inkubieren dieser Blutprobe mit einem in den Ansprüchen 1 bis 4 verwendeten Antikörper,
- Quantifizieren der an Zellen gebundenen Antikörper.

13. Verfahren zur Bereitstellung von hämatopoetischen Vorläuferzellen, die in Mastzellen oder Basophile differenzieren können, mit den Schritten:
- Bereitstellen von aus einem Organismus isolierten Knochenmarkzellen,
- Inkubieren dieser Knochenmarkzellen mit einem in den Ansprüchen 1 bis 4 verwendeten Antikörper,
- Isolieren der antikörpergebundenen Zellen mit üblichen Verfahren, insbesondere FACS oder MACS.

## Claims

1. The use of an antibody for the detection and/or quantification and/or isolation of basophils and/or mast cells and/or of the precursor cells of basophils and/or mast cells and/or of a surface structure of said cells, **characterized in that** binding of the antibody occurs to the surface structure PDNP3 (phosphodiestrase/nucleotid-pyrophosphatase-ectoenzyme) of the cells to which can bind the antibody with the designation 97A6, produced and released by hybridoma cells that were deposited in accordance with the Budapest Treaty on February 12, 1997 under number DSM ACC 2297 at Deutsche Sammlung von Mikroorganismen und Zellkulturen GmbH (DSMZ).

2. The use as defined in Claim 1, **characterized in that** the antibody used is a monoclonal antibody.

3. The use as defined in Claim 1 or Claim 2, **characterized in that** the antibody essentially does not interact with immunoglobulins of the IgE class.

4. The use as defined in any of Claims 1 through 3, **characterized in that** antibody 97A6 itself is used for detection and/or isolation.

5. The use as defined in any of Claims 1 through 4 in conjunction with the analysis of hematopoiesis.

6. The use as defined in any of Claims 1 through 5 in conjunction with the analysis of patient samples, in particular of tissue biopsies, bone marrow biopsies, and/or blood samples.

7. The use as defined in any of Claims 1 through 6 in conjunction with the diagnostic classification of tumors, in particular of leukemias.

8. The use as defined in any of Claims 1 through 7, **characterized in that** the antibody is joined to a marker, in particular to a fluorescent marker.

9. The use as defined in any of Claims 1 through 8, **characterized in that** the detection of bound antibodies is accomplished by way of a usual immunological detection method, in particular ELISA, or FACS analysis.

10. The use as defined in any of Claims 1 through 9 for detecting and/or quantifying activated basophils.

11. The use as defined in any of Claims 1 through 10 for determination of the extent to which basophils are activated.

12. A method for investigating allergies, comprising the steps:
- incubating a blood sample with an agent that is suspected of triggering an allergic reaction;
- incubating said blood sample with an antibody used in Claims 1 through 4;
- quantifying the antibodies bound to cells.

13. A method for providing hematopoietic precursor cells that can differentiate into mast cells or basophils, comprising the steps:
- isolation and provision of bone marrow cells from an organism;
- incubation of said bone marrow cells with an antibody used in Claims 1 through 4;
- isolation of the antibody-bound cells using usual methods, in particular FACS or MACS.

## Revendications

1. Utilisation d'un anticorps pour la détection et/ou la quantification et/ou l'isolement de basophiles et/ou de mastocytes et/ou de précurseurs des basophiles et/ou mastocytes et/ou d'une structure superficielle de ces cellules, **caractérisée en ce qu'**il se produit une liaison de l'anticorps avec la structure superficielle PDNP3 (ectoenzyme phosphodiestérase/nucléotide-pyrophosphatase) des cellules, à laquelle peut se lier l'anticorps baptisé 97A6, qui est produit et libéré à partir de cellules d'hybridome, qui ont été déposées le 12.02.1997 sous la référence DSM ACC 2297 auprès de la Sammlung von Mikroorganismen und Zellkulturen GmbH, conformément au Traité de Budapest.

2. Utilisation selon la revendication 1, **caractérisée en ce que** l'anticorps utilisé est un anticorps monoclonal.

3. Utilisation selon la revendication 1 ou la revendication 2, **caractérisée en ce que** l'anticorps n'interagit pas fondamentalement avec les immunoglobulines de classe IgE.

4. Utilisation selon l'une des revendications 1 à 3, **caractérisée en ce que** l'anticorps 97A6 est utilisé lui-même pour la détection et/ou l'isolement.

5. Utilisation selon l'une des revendications 1 à 4 en relation avec une analyse de l'hématopoïèse.

6. Utilisation selon l'une des revendications 1 à 5 en relation avec une analyse d'échantillons de patients, en particulier de biopsie tissulaire, de biopsie de moelle osseuse et/ou d'échantillons sanguins.

7. Utilisation selon l'une des revendications 1 à 6 en relation avec une classification diagnostique de tumeurs, en particulier de leucémies.

8. Utilisation selon l'une des revendications 1 à 7, **caractérisée en ce que** l'anticorps est lié à un marqueur, en particulier un marqueur fluorescent.

9. Utilisation selon l'une des revendications 1 à 8, **caractérisée en ce que** t'existence d'anticorps liés est prouvée par un procédé de détection immunologique habituel, en particulier une analyse ELISA ou FACS.

10. Utilisation selon l'une des revendications 1 à 9 pour prouver l'existence et/ou quantifier des basophiles activés.

11. Utilisation selon l'une des revendications 1 à 10 pour la détermination de l'importance de l'activation des basophiles.

12. Procédé pour l'examen d'allergies, comportant les étapes suivantes :
- incubation d'un échantillon sanguin avec un agent que l'on suspecte de provoquer une réaction allergique,
- incubation de cet échantillon sanguin avec un anticorps utilisé selon l'une des revendications 1 à 4,
- quantification des anticorps liés aux cellules.

13. Procédé de préparation de précurseurs hématopoïétiques, qui peuvent se différencier dans des mastocytes ou des basophiles, comportant les étapes suivantes :
- préparation de cellules de moelle osseuse isolées à partir d'un organisme,
- incubation de ces cellules de moelle osseuse avec un anticorps utilisé selon l'une des revendications 1 à 4,
- isolement des cellules liées aux anticorps selon un procédé habituel, en particulier un trieur de cellules à fluorescence (FACS) ou un trieur de cellules à billes magnétiques (MACS).
